# EUROPEAN PATENT SPECIFICATION

(11) **EP 2 787 072 B1**
(45) Date of publication and mention of the grant of the patent: **05.04.2017**
(21) Application number: 13005707.8
(22) Date of filing: 07.12.2013
(51) Int. Cl.: C12N 1/20, C12P 19/04, A61L 27/20, A61K 47/38, C08L 1/02

(54) **A method of production of a stable composite of bacterial bionanocellulose with perforated metal or polymeric material, designed for tissues reconstruction**
Die Herstellungsmethode eines für Geweberekonstruktion bestimmten stabilen Verbundstoffes der bakteriellen Bionanozellulose mit perforiertem Polymer- oder Metallmaterial
La méthode de la production du composite solide de bionanocellulose bactérienne avec un matériau perforé polymère ou métallique, destiné à la reconstruction des tissus

(30) Priority: 05.04.2013 PL 40343813
(43) Date of publication of application: 08.10.2014
(73) Proprietor: Politechnika Lodzka, 90-924 Lodz (PL)
(72) Inventor: Kolodziejczyk, Marek, 92-511 Lódz (PL); Ludwicka, Karolina, 93-165 Lódz (PL); Pankiewicz, Teresa, 93-347 Lódz (PL); Bielecki, Stanislaw, 92-117 Lódx (PL)
(74) Representative: Kaczur-Kaczynska, Ewa

(56) References cited:
- WO-A1-97/05271
- US-A1- 2012 094 334
- DANUTA CIECHANSKA ET AL: "Surface Biomodification of Surgical Meshes Intended for Hernia Repair", FIBRES & TEXTILES IN EASTERN EUROPE, vol. 20, no. 6B, 1 November 2012 (2012-11-01), pages 6-107, XP055128274, ISSN: 1230-3666

## Description

The subject of invention is the method of production of a stable composite of bacterial bionanocellulose with perforated metal or polymeric material, designed for the reconstruction of soft and hard tissues, in stationary culture of *Gluconacetobacter xylinus.*

Bacterial bionanocellulose (BNC) in recent years has been subjected to *numerous in vivo* analyses in different applications. Many publications confirm that BNC as a biomaterial nondegradable inside the body fulfils its functions by displaying an adequate, high hydrophilicity, lack of cytotoxicity, great biocompatibility and stability in a wide range of temperatures and pH. It was demonstrated to be effective in the regeneration of skin, cartilage, in reconstruction of small blood vessels and as a drug carrier. Bionanocellulose material is being applied also as a biocompatible cover for other polymers, mainly synthetic ones, widely used in medicine. It not only improves their biocompatibility and acceptance by host organism, but also lowers their roughness, preventing from the irritation of surrounding tissues and internal organs and tissues adhesions to the rough structure of such polymers.

Up to date there were described the methods of integration of synthetic meshes with BNC *in situ* horizontally during the cultivation process.

In patent application WO/2011/038373 the authors described a bioreactor enabling the continuous spraying of culture medium onto the materials placed at the bottom of the reactor, which is to facilitate their uniform overgrowing with cellulose layers.

In Advanced Materials Research (2008, vol. 47-50, p. 1371-1374) it is proposed to treat the polypropylene mesh with plasma before inserting it horizontally to the culture medium. The goal of such treatment is to modify the surface of this hydrophobic polymeric material by enhancing its hydrophilicity and subsequently to improve its overgrowth with hydrophilic cellulose.

From Fibers & Textiles in Eastern Europe (2012, 20:6B (96): 107-114) there is a known method of BNC covering a polypropylene surgical mesh placed at the bottom of bioreactor, by applying the culture medium of *Acetobacter xylinus* enriched in chitosan. The cultivation is performed with simultaneous temporary reversal of the mesh in the bioreactor, so as to cover both its sites with bionanocellulose.

All the above described methods of BNC composites preparation apply horizontal cultivation, where the overgrown material is laid down on the bottom of the bioreactor. For that reason they are characterised by the following inconveniences:
- unusual difficulty in obtaining a stable, difficult to break connection of cellulose with the overgrown material,
- difficulty in bacterial movement and penetration of the structure of such horizontally placed materials, despite of their porous, perforated construction,
- placing the overgrown material at the bottom of the cultivation dish or at its any level perpendicular to the surface of the medium makes impossible the free movement of bacteria vertically in the culture medium, resulting in incorrect, unstable connection of cellulosic membrane with the material or its complete absence.

The method of production of a stable composite of bacterial bionanocellulose with perforated metal or polymeric material, designed for the reconstruction of soft and hard tissues, that involves the fixing of a sterile, perforated polymeric or metallic material in a cultivation medium of *Guconacetobcter xylinus,* composed of 20 parts by weight of glucose, 5 parts by weight of yeast extract, 5 parts by weight of peptone, 2.5 parts by weight of MgSO₄ x 7H₂O, 2.7 parts by weight of Na₂HPO₄, 1.15 parts by weight of citric acid and 10 parts by weight of ethanol, dissolved in 1000 parts by weight of distilled water, inoculated with bacteria pre-cultivated in the same medium at the temperature of 27-33° C, and further conducting of stationary culture at 27-33° C until the total connection of cellulose membrane with a polymeric or metallic material on its whole surface, **according to the invention is characterized** by the fact, that the cultivation is performed in a bioreactor with the walls inclined at the angle of 90-30° in relation to the base of bioreactor, wherein the perforated polymeric or metallic material, with the thickness up to 0.5 cm and single pores surface above 0.5 mm², used to be overgrown with cellulose, is vertically hanged in a bioreactor, so that material's lower edge is suspended in a culture medium for the first 2-3 days, i.e. until the first layers of cellulose are formed, and in the next days it is successively dropped down into the medium until it is totally covered with cellulose membrane. The overgrown material is further taken off from the bioreactor, excised from the excess of the membrane, purified, pressed until the proper thickness, packed and sterilized. There is one or more polymeric or metallic perforated materials of different thickness, pores sizes and elasticity, that are fixed vertically in the bioreactor at a distance of 0.5-10 cm from each other and are overgrown simultaneously by cellulose membrane.

According to the method described the invention provides a composite of perforated metal or polymeric material with overgrowing it hydrophilic, fibrous structure of bionanocellulose, which is a qualitatively new, showing no roughness biocompatible product to be used in reconstructive surgery.

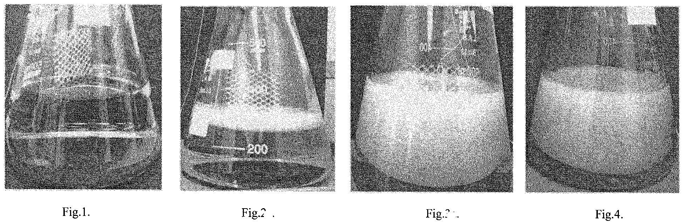

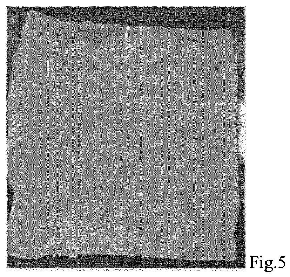

The subject of invention is illustrated by the examples described below, together with the figures, where fig. 1-4 demonstrate the subsequent stages of composite production and fig. 5 shows a ready to use product.

### Example 1.

An inoculum of *Gluconacetobacter xylinus* was cultivated in a medium composed of 20 parts by weight of glucose, 5 parts by weight of yeast extract, 5 parts by weight of peptone, 2.5 parts by weight of MgSO₄ x 7H₂O, 2.7 parts by weight of Na₂HPO₄,1.15 parts by weight of citric acid and 10 parts by weight of ethanol, dissolved in 1000 parts by weight of distilled water, at the temperature of 27-33° C. This inoculum served for inoculation of a culture medium of the same composition, contained in a bioreactor, with the walls inclined at the angle of 75° in relation to its base. Next, a sterile, monofilament, perforated polypropylene mesh of two-directional elasticity and size of 4 x 4 cm, thickness 0.45 mm, and single pore size of 2 x 2.5 mm, was vertically hanged in a bioreactor, so that the material's lower edge was suspended in a culture medium for the first 2 days, i.e. until the first layers of cellulose were formed. In the next days the mesh was successively dropped down into the medium until it was totally covered with cellulose membrane, i.e. up to approx. 14 days of cultivation. The culture was kept in 30° C and all the procedures were performed in sterile conditions. The overgrown material was further taken off from the bioreactor, excised from the excess of the membrane, pressed until the thickness of 3 mm, packed and sterilized.

The final composite was white in colour, with neutral pH and well visible mesh overgrown with cellulose.

### Example 2.

Following the procedures described in the previous example, the culture medium with *Gluconacetobacter xylinus* was prepared. In the bioreactor 3 perforated meshes of the same parameters as described in example 1 were hanged at a distance of 1 cm one from another. The cultivation was conducted analogously to the example 1. The produced composited, i.e. meshes overgrown with bionanocellulose were taken off from the bioreactor, excised from the excess of the membrane, pressed until the thickness of 3 mm, packed and sterilized.

## Claims

1. The method of production of a stable composite of bacterial bionanocellulose with perforated metal or polymeric material, designed for the reconstruction of soft and hard tissues, involving the vertical fixing of a sterile, perforated polymeric or metallic material in a cultivation medium of *Guconacetobcter xylinus,* composed of 20 parts by weight of glucose, 5 parts by weight of yeast extract, 5 parts by weight of peptone, 2.5 parts by weight of MgSO₄ x 7H₂O, 2.7 parts by weight of Na₂HPO₄, 1.15 parts by weight of citric acid and 10 parts by weight of ethanol, dissolved in 1000 parts by weight of distilled water, inoculated with bacteria precultivated in the same medium at the temperature 27-33° C, and further conducting of stationary culture at 27-33° C until the total connection of cellulose membrane with a polymeric or metallic material on its whole surface, is **characterized by the fact,** that the cultivation is performed in a bioreactor with the walls inclined at the angle of 90-30° in relation to the base of bioreactor, wherein the perforated polymeric or metallic material, with the thickness up to 0.5 cm and single pores surface above 0.5 mm², used to be overgrown with cellulose, is vertically hanged in a bioreactor, so that material's lower edge is suspended in a culture medium until the first layers of cellulose are formed, and in the next days it is successively dropped down into the medium until this perforated material is completely overgrown with bionanocellulose, the overgrown material is further taken off from the bioreactor, excised from the excess of the membrane, pressed until the proper thickness, packed and sterilized.

2. The method claimed in the claim 1, **is characterized by the fact,** that one or more polymeric or metallic perforated materials of different thickness, pores sizes and elasticity are fixed vertically in the bioreactor at a distance of 0.5-10 cm from each other and are overgrown simultaneously by cellulose membrane.

## Patentansprüche

1. Verfahren zur Herstellung eines stabilen Verbundes aus bakterieller Bionanozellulose mit perforiertem polymerischen oder metallischen Material, vorgesehen zur Rekonstruktion von weichem und hartem Gewebe, beruhend auf der Einbettung des vertikalen, sterilen, perforierten Polymermaterials oder Metallmaterials im Nährboden zur Zucht des Bakterienstamms *Gluconacetobacter xylinus ,* mit 20 Anteilen an Gewichtsanteilen von Glukose, 5 Gewichtsanteilen an Hefeextrakt, 5 Gewichtsanteilen an Pepton, 2,5 Anteilen an MgSO₄ x 7 H₂O, 2,7 Anteilen an Na₂HPO₄, 1,15 Anteilen an Zitronensäure, 10 Anteilen an Ethanol, bis zu 1.000 Anteilen an destilliertem Wasser, beimpft mit dem Inokulum der Bakterien, gezüchtet auf einer inokularen Basis in derselben Zusammensetzung in einer Temperatur von 27-33° C und einer stationären Züchtung dieser Bakterien bei einer Temperatur von 27-33° C bis zur Verbindung des polymeren oder metallischen Materials mit der Bionanozellulose auf der gesamten Oberfläche, **dadurch gekennzeichnet, dass** die Zucht der Bakterien in einem Bioreaktor mit Wänden einer Neigung von 90 - 30 ° im Verhältnis zur Basis vollzogen wird, wobei das zur Verbindung mit der Bionanozellulose perforierte Polymer- oder Metallmaterial mit einer Stärke von 0,5 cm und einer Porengröße von 0,5 mm² in dem Bioreaktor vertikal so aufgehängt wird, dass bis zur Bildung der ersten Schichten von Bionanozellulose nur der untere Rand in das Medium eingetaucht ist, wobei dann in den folgenden Tagen der Züchtung das Material stufenweise in das Medium abgelassen wird, bis es auf der gesamten Fläche durch Bionanozellulose überlagert wird, woraufhin das von der Bionanozellulose überlagerte Material aus dem Bioreaktor herausgenommen wird, vom Übermaß an Hautbildung befreit wird und in die entsprechende Dicke komprimiert, gepackt und sterilisiert wird.

2. Verfahren gemäß Patentanspruch 1, **gekennzeichnet dadurch, dass** die aufgezogene Bionanozellulose ein Material oder mehrere Materialien verschiedener Dicke, Maße, Porosität und Festigkeit ergibt, die im Bioreaktor in einem Abstand von 0,5 - 10 cm angebracht sind.

## Revendications

1. Procédé permettant de produire un composite permanent de la bionanocellulose bactérienne avec le matériau perforé polymèrique ou métallique, destiné à la reconstruction des tissus mous et durs, se reposant sur un matériau stérile, perforé, polymèrique ou métallique, disposé verticalement, dans le milieu de culture de la souche bactérienne *Gluconacetobacter xylinus,* contenant 20 parties (en poids) de glucose, 5 parties (en poids) de levure, 5 parties (en poids) de peptone, 2,5 parties (en poids) de MgSO₄ x 7 H₂O, 2, 7 parties (en poids) de Na₂HPO₄, 1,5 parties (en poids) d'acide citrique, 10 parties (en poids) d'éthanol, à 100 parties (en poids) d'eau distillée, greffée d'inoculum de ces bactéries, élevées sur la base d'inoculum, de même composition, dans la température de 27-33 °C, jusqu'au fusionnement du matériau polymèrique ou métallique avec le film de la bionanocellulose sur toute la surface. Il est important que le milieu de culture de la souche bactérienne est mené dans le bioréacteur avec des parois sous un angle de 90-30 ° par rapport à la base, et le matériau perforé polymèrique ou métallique, destiné au fusionnement avec le film de la bionanocellulose, ayant une épaisseur de 0,5 cm et une taille de pores supérieure à 0,5 mm2, est suspendu vérticalement dans le bioréacteur jusuq'à la création de premières couches de la bionanocellulose, que le bord inférieur de celui-ci est immergée sous la surface du support, puis dans les jours suivants de matériel de culture est progressivement réduit dans le milieu jusuq'à son surpassement sur toute la surface, bionanocellulose, puis le matériau surpassé par le film de la bionanocellulose est enlevé du bioréacteur, découpé de de l'excès de film, comprimé à l'épaisseur souhaitée, conditionné et stérilisé.

2. Procédé, selon la restriction 1. Il est à noter que le surpassement du film de la bionanocellulose est soumis un ou plusieurs matériaux à la fois, 'épaisseurs différentes, le grammage, la porosité et la dureté sont disposés verticalement dans le fermenteur odle-porteurs de 0,5 - 10 cm les unes des autres.
